# EUROPEAN PATENT SPECIFICATION

(11) **EP 2 098 232 B1**
(45) Date of publication and mention of the grant of the patent: **05.06.2013**
(21) Application number: 07832913.3
(22) Date of filing: 30.11.2007
(51) Int. Cl.: A61K 31/445, A61K 9/70, A61K 47/02, A61K 47/14, A61K 47/32, A61P 25/28

(54) **PERCUTANEOUS ABSORPTION PREPARATION**
ZUBEREITUNG MIT PERKUTANER ABSORPTION
PRÉPARATION D'ABSORPTION PERCUTANÉE

(30) Priority: 01.12.2006 JP 2006325104; 01.12.2006 US 861965 P
(43) Date of publication of application: 09.09.2009
(73) Proprietor: NITTO DENKO CORPORATION, Ibaraki-shi Osaka 567-8680 (JP); Eisai R&D Management Co., Ltd., Tokyo 112-8088 (JP)
(72) Inventor: HANATANI, Akinori, Ibaraki-shi Osaka 567-8680 (JP); WASHIRO, Satoko, Ibaraki-shi Osaka 567-8680 (JP); AKEMI, Hitoshi, Ibaraki-shi Osaka 567-8680 (JP)
(74) Representative: HOFFMANN EITLE
(86) International application number: PCT/JP2007/073248
(87) International publication number: WO 2008/066179

(56) References cited:
- EP-A1- 1 086 706
- EP-A1- 1 437 130
- EP-A1- 1 541 177
- WO-A1-2006/070930
- WO-A1-2006/082728
- WO-A1-2007/129712
- JP-A- 2003 062 058
- JP-A- 2004 010 525
- JP-A- 2007 016 019
- MORIEARTY P L ET AL: "Transdermal patch delivery of acetylcholinesterase inhibitors", METHODS AND FINDINGS IN EXPERIMENTAL AND CLINICAL PHARMACOLOGY, PROUS, BARCELONA, ES, vol. 15, no. 6, 1 January 1993 (1993-01-01), pages 407-412, XP009120198, ISSN: 0379-0355

## Description

### [Field of the Invention]

The present invention relates to a percutaneously absorbable preparation comprising 2-[(1-benzyl-4-piperidinyl)methyl]-5,6-dimethoxyindan-1-on (hereinafter called "donepezil") and/or its hydrochloride 2-[(1-benzyl-4-piperidinyl)methyl]-5,6-dimethoxyindan-1-on monohydrochloride (hereinafter called "donepezil hydrochloride"). Specifically, this is a percutaneously absorbable preparation to be applied as a patch to the skin surface in order to continuously administer this drug to a living body through the skin.

### [Background of the Invention]

The basic drug donepezil, or its hydrochloride donepezil hydrochloride, has acetylcholine esterase inhibitory action and is used against Alzheimer's dementia. Alzheimer's dementia patients are usually elderly, and often have difficulty swallowing oral dosage. In some cases it may also be difficult to administer oral dosage to patients with advanced symptoms of Alzheimer's dementia. In these cases, non-oral administration is useful.

Percutaneously absorbable preparations using donepezil or donepezil hydrochloride are already known, and for example a preparation for percutaneous use (such as an ointment or skin patch) containing donepezil hydrochloride is proposed in Japanese Patent Application Laid-open No. H11-315016. WO 2003/032960 also proposes a percutaneously absorbable dementia treatment preparation containing donepezil or its hydrochloride in a pressure-sensitive adhesive composition, and describes that when using a hydrochloride of donepezil, a satisfactory skin permeation rate can be obtained by including an acetic acid salt in the pressure-sensitive adhesive composition.

In general a short period of administration is preferred when administering a drug for a disease, but in actuality long-term administration is often necessary. When a percutaneously absorbable preparation is used for such long-term administration, it must be applied and peeled off almost every day. Because movable parts of the body are desirably avoided as application sites, moreover, there is a limit on the available application sites. Consequently, from the standpoint of repeated application, a percutaneously absorbable preparation should be as gentle to the skin as possible, and should cause little corneous damage and other irritation of the skin surface or in other words have low dermal irritancy. However, neither Japanese Patent Application Laid-open No. H11-315016 nor WO 2003/032960 discusses the problem of skin feel or irritancy when the percutaneously absorbable application is applied to the skin, or measures to combat this problem.

Methods for obtaining low-irritancy percutaneously absorbable preparations include a method of reducing the skin adhesiveness to a reasonable level by adjusting the composition of the pressure-sensitive adhesive itself, and a method of including a liquid component in the pressure-sensitive adhesive layer to produce a gel, thereby giving a softer feeling to the pressure-sensitive adhesive layer. To prepare this gel, a method has been used whereby cohesiveness is improved by crosslinking the pressure-sensitive adhesive, and the pressure-sensitive adhesive layer is made to hold a compatible liquid component.

Any crosslinking agent can be used to crosslink the pressure-sensitive adhesive in preparing this gel.

In recent years, however, a percutaneously absorbable preparation using a gel comprising a combination of a particular kind of basic drug and a particular kind of crosslinking agent reportedly declines in cohesiveness of the pressure sensitive adhesive layer during wear to humans, leading to cohesive failure when it is peeled off.

To cope with such problems, a method has been proposed of further developing the effect of the crosslinking agent by adding a polyol compound together with the crosslinking agent (Japanese Patent Application Laid-open No. 2003-62058), as well as a method whereby this problem is avoided by designing a placebo layer for the skin adhesion layer using a crosslinking agent that is resistant to cohesive failure when peeled, including the basic drug in the layer under this, and laminating a pressure-sensitive adhesive layer crosslinked with a crosslinking agent that resists loss of cohesiveness even in the presence of the drug (Japanese Patent Application Laid-open No. 2004-10525).

However, in the former method (Japanese Patent Application Laid-open No. 2003-62058), the hydrophilic nature of the polyol compound may possibly limit the concentration of the uniform dissolution of the compound to about 5 wt% at most, when using the pressure-sensitive adhesive layer in a hydrophobic environment. A higher concentration may lead to blooming from the pressure-sensitive adhesive layer. Because of this, when a relatively large amount of basic drug is contained in the pressure-sensitive adhesive layer, the content of the polyol compound uniformly dissolved in the pressure-sensitive adhesive layer is further reduced, and may not be sufficient to prevent cohesive failure of the pressure-sensitive adhesive layer. The content of the polyol compound in the pressure-sensitive adhesive layer could be increased, but this would reduce the content of the pressure-sensitive adhesive in the pressure-sensitive adhesive layer, potentially detracting from adhesion. The second method (Japanese Patent Application Laid-open No. 2004-10525) succeeds in controlling cohesive failure of the patch surface during peeling, but there may be cohesive failure of the edge when the sides of the edge of the percutaneously absorbable preparation contact the skin during use,.
[Patent Reference 1] JP-A-H11-315016
[Patent Reference 2] WO 2003/032960 A
[Patent Reference 3] JP-A-2003-62058
[Patent Reference 4] JP-A-2004-10525

### [Disclosure of the Invention]

### [Problems To Be Solved by the Invention]

Under these circumstances, it is an object of the present invention to provide a percutaneously absorbable preparation for percutaneous absorption of donepezil and/or donepezil hydrochloride which is a percutaneously absorbable preparation having good adhesiveness and cohesiveness, and which is resistant to loss of cohesiveness of the pressure-sensitive adhesive layer when attached to the skin and resists adhesive transfer due to cohesive failure when removed by peeling.

It is also an object to provide a percutaneously absorbable preparation for percutaneous absorption of donepezil and/or donepezil hydrochloride which is a percutaneously absorbable preparation having good adhesiveness and cohesiveness and excellent skin feel, which provides low skin irritancy during peeling, and which is resistant to loss of cohesiveness of the pressure-sensitive adhesive layer when attached to the skin and resists adhesive transfer due to cohesive failure when removed by peeling.

### [Means for Solving the Problems]

As a result of exhaustive research aimed at solving these problems, the inventors arrived at the present invention upon discovering that loss of cohesiveness of the pressure-sensitive adhesive layer during attachment to the skin could be prevented and adhesive transfer avoided during removal by peeling by including a metal chloride in a crosslinked pressure-sensitive adhesive layer comprising donepezil and/or donepezil hydrochloride. Accordingly, the present invention is as follows.

(1) A percutaneously absorbable preparation comprising 2-[(1-benzyl-4-piperidinyl)methyl]-5,6-dimethoxyindan-1-on and/or its hydrochloride and a metal chloride in a pressure-sensitive adhesive layer, wherein the pressure-sensitive adhesive layer is crosslinked.
(2) The percutaneously absorbable preparation according to (1) above, wherein the pressure sensitive adhesive layer is crosslinked using a crosslinking agent.
(3) The percutaneously absorbable preparation according to (2) above, wherein the crosslinking agent is a metal alcoholate or metal chelate compound.
(4) The percutaneously absorbable preparation according to any one of (1) through (3) above, wherein the pressure-sensitive adhesive is an acrylic pressure-sensitive adhesive.
(5) The percutaneously absorbable preparation according to any one of (1) through (4), wherein the pressure-sensitive adhesive layer further comprises a liquid plasticizer.
(6) The percutaneously absorbable preparation according to (5) above, wherein the liquid plasticizer is a fatty acid alkyl ester produced by reacting a higher fatty acid having 12 to 16 carbon atoms with a lower monohydric alcohol having 1 to 4 carbon atoms.
(7) The percutaneously absorbable preparation according to any one of (1) through (6) above, wherein the metal chloride is at least one species selected from the group consisting of sodium chloride, calcium chloride, aluminum chloride, stannous chloride and ferric chloride.
(8) The percutaneously absorbable preparation according to any one of (1) through (6) above, wherein the metal chloride is sodium chloride.
(9) The percutaneously absorbable preparation according to (1) above, wherein the metal chloride is a salt produced by neutralizing a hydrochloride of 2-[(1-benzyl-4-piperidinyl)methyl]-5,6-dimethoxyindan-1-on with an inorganic base containing a metal.
(10) The percutaneously absorbable preparation according to (9) above, wherein the inorganic base containing a metal is an inorganic base containing an alkali metal or alkaline earth metal.

### [Advantageous Effects of the Invention]

According to the percutaneously absorbable preparation of the present invention, decline of cohesiveness of the pressure-sensitive adhesive layer during application to the skin is controlled by including a metal chloride in a crosslinked adhesive layer comprising donepezil and/or donepezil hydrochloride, resulting in a stable percutaneously absorbable preparation which resists adhesive transfer due to cohesive failure when peeled.

In particular, in a preparation of a type comprising a liquid plasticizer in a pressure-sensitive adhesive layer, since decline of cohesiveness of the pressure-sensitive adhesive layer is controlled during wear to the skin and since the liquid plasticizer is held stably in the pressure-sensitive adhesive layer, it is possible to obtain a stable percutaneously absorbable preparation with low skin irritancy which has excellent skin feel and little skin irritancy when peeled and which resists adhesive transfer due to cohesive failure when peeled.

### [Best Mode for Carrying Out the Invention]

The present invention is explained in greater detail below.
The percutaneously absorbable preparation of the present invention is for percutaneous absorption of donepezil and/or donepezil hydrochloride (hereinafter, "donepezil and/or donepezil hydrochloride" may be abbreviated as "donepezil "), and comprises donepezil and/or donepezil hydrochloride and a metal chloride in a pressure-sensitive adhesive layer that has undergone a crosslinking process. From the standpoint of percutaneous absorbability, the pressure-sensitive adhesive layer should preferably contain at least donepezil.

The percutaneously absorbable preparation of the present invention can be used as an Alzheimer's dementia drug. Examples of other uses include for cerebrovascular dementia treatment, and, migraine prevention.

In the percutaneously absorbable preparation of the present invention, the content of donepezil and/or donepezil hydrochloride in the pressure-sensitive adhesive layer is normally 1 to 30 wt% or preferably 3 to 20 wt% of the total weight of the pressure-sensitive adhesive layer. If the content is less than 1 wt% an effective dose for therapy may not be released, while more than 30 wt% is uneconomical without providing any further therapeutic benefits.

The pressure-sensitive adhesive forming the pressure-sensitive adhesive layer in the percutaneously absorbable preparation of the present invention is not particularly limited as long as it can be crosslinked, but examples include acrylic pressure-sensitive adhesives; silicone rubber, polyisoprene rubber, polyisobutylene rubber, styrenebutadiene rubber, styrene-isoprene-styrene block copolymer rubber, styrene-butadienestyrene block copolymer rubber and other rubber-based pressure-sensitive adhesives; silicone pressure-sensitive adhesives; and polyvinyl alcohol, polyvinyl alkyl ether, polyvinyl acetate and other vinyl polymer pressure-sensitive adhesives.

It is essential in the percutaneously absorbable preparation of the present invention that the pressure sensitive adhesive layer is crosslinked through for example a crosslinking treatment thereof The treatment may be accomplished by known chemical or physical crosslinking processes (such as crosslinking by exposure to ultraviolet rays or gamma-rays or other electron rays in the case of physical crosslinking or crosslinking by addition of a crosslinking agent in the case of chemical crosslinking). It is therefore desirable that the pressure-sensitive adhesive have introduced functional groups capable of contributing to a crosslinking reaction, such as hydroxyl groups, carboxyl groups, vinyl groups or the like, and these functional groups capable of contributing to a crosslinking reaction can be introduced into the pressure-sensitive adhesive by known methods. For example, during synthesis of the polymer for the pressure-sensitive adhesive a monomer having hydroxyl groups such as hydroxyethyl (meth)acrylate or a monomer having carboxyl groups such as acrylic acid or maleic acid can be added and copolymerized. Crosslinking treatment of the pressure-sensitive adhesive layer can also be accomplished by adding and copolymerizing a monomer such as divinyl benzene, ethylene glycol dimethacrylate or the like having two or more vinyl groups during synthesis of the polymer for the pressure-sensitive adhesive to thereby cause intermolecular or intramolecular crosslinking during the copolymerization reaction.

Of those mentioned above, the pressure-sensitive adhesive of the present invention is preferably an acrylic pressure-sensitive adhesive from the standpoint of ease of crosslinking and good skin adhesiveness of the percutaneously absorbable preparation.

The acrylic pressure-sensitive adhesive of the present invention is normally an acrylic pressure-sensitive adhesive comprising a (meth)acrylic acid alkyl ester, and is preferably an acrylic pressure-sensitive adhesive having a (meth)acrylic acid alkyl ester as the principal component (principal constituent unit), or more preferably a copolymer of a (meth)acrylic acid alkyl ester as the principal component (first monomer component) with a vinyl monomer having functional groups capable of contributing to a crosslinking reaction (second monomer component), and a copolymer copolymerized with another monomer (third monomer component) in addition to these is especially desirable from the standpoint of ease of crosslinking treatment, adhesiveness to human skin, and ease of drug dissolution.

Examples of the (meth)acrylic acid alkyl ester (first monomer component) include (meth)acrylic acid alkyl esters and the like wherein the alkyl group is a straight-chain, branched or cyclic alkyl group with 1 to 18 carbon atoms (such as a methyl, ethyl, propyl, butyl, pentyl, hexyl, cyclohexyl, heptyl, octyl, 2-ethylhexyl, nonyl, decyl, undecyl, dodecyl, tridecyl, etc.), and a (meth)acrylic acid alkyl ester wherein the alkyl group is a straight-chain, branched or cyclic alkyl group with 4 to 18 carbon atoms (such as a butyl, pentyl, hexyl, cyclohexyl, heptyl, octyl, 2-ethylhexyl, nonyl, decyl, undecyl, dodecyl or tridecyl) is preferred. Since a monomer component that lowers the glass transition temperature of the polymer is particularly desirable for contributing room-temperature adhesiveness, a (meth)acrylic acid alkyl ester wherein the alkyl group is a straight-chain, branched or cyclic alkyl group with 4 to 8 carbon atoms (such as a butyl, pentyl, hexyl, cyclohexyl, heptyl, octyl, 2-ethylhexyl or the like or preferably a butyl, 2-ethylhexyl or cyclohexyl or most preferably a 2-ethylhexyl) is more preferred. Specifically, butyl acrylate, 2-ethylhexyl acrylate, 2-ethylhexyl methacrylate, cyclohexyl acrylate, cyclohexyl methacrylate or the like is desirable, and of these, 2-ethylhexyl acrylate is particularly desirable. One such (meth)acrylic acid alkyl ester (first monomer component) may be used, or two or more may be used in combination.

In the vinyl monomer (second monomer component) having functional groups capable of contributing to the crosslinking reaction, the functional groups capable of contributing to the crosslinking reaction may be hydroxyl groups, carboxyl groups, or vinyl groups, and hydroxyl groups and carboxyl groups are preferred. Specific examples of this monomer (second monomer component) include hydroxyethyl (meth)acrylate esters, hydroxypropyl (meth)acrylate esters, (meth)acrylic acid, itaconic acid, maleic acid, maleic anhydride, mesaconic acid, citraconic acid, glutaconic acid and the like. Of these, acrylic acid, methacrylic acid or a hydroxyethyl acrylate ester (particularly 2-hydroxyethyl acrylate) is preferred from the standpoint of availability, and acrylic acid is particularly desirable. One such monomer (second monomer component) may be used, or two or more may be used in combination.

The aforementioned other monomer (third monomer component) is used primarily to adjust the cohesiveness of the pressure-sensitive adhesive layer and to adjust the solubility or release properties of the donepezil. Examples of this monomer (third monomer component) include vinyl acetate, vinyl propionate and other vinyl esters; methyl vinyl ether, ethyl vinyl ether and other vinyl ethers; N-vinyl-2-pyrrolidone, N-vinyl caprolactam and other vinyl amides; methoxyethyl (meth)acrylate ester, ethoxyethyl (meth)acrylate ester, tetrahydrofurfuryl (meth)acrylate ester and other alkoxy (meth)acrylate esters; hydroxypropyl (meth)acrylate, α-hydroxymethyl acrylate and other hydroxyl-containing monomers (which do not provide crosslinking points because they are used as the third monomer component); (meth)acrylamide, dimethyl (meth)acrylamide, N-butyl (meth)acrylamide, N-methylol (meth)acrylamide and other (meth)acrylic acid derivatives with amide groups; aminoethyl (meth)acrylate ester, dimethylamino (meth)acrylate ester, t-butylaminoethyl (meth)acrylate ester and other aminoalkyl (meth)acrylate esters; methoxyethylene glycol (meth)acrylate ester, methoxydiethylene glycol (meth)acrylate ester, methoxypolyethylene glycol (meth)acrylate ester, methoxypolypropylene glycol (meth)acrylate ester and other alkoxyalkylene glycol (meth)acrylate esters; (meth)acrylonitrile; styrene sulfonic acid, allyl sulfonic acid, sulfopropyl (meth)acrylate, (meth)acryloyl oxynaphthalene sulfonic acid, acrylamide methyl sulfonic acid and other monomers having sulfonic acid; and vinyl piperidone, vinyl pyrimidine, vinyl piperazine, vinyl pyrrole, vinyl imidazole, vinyl oxazole, vinyl morpholine and other vinyl group-containing monomers. Of these, a vinyl ester or vinyl amide is preferred, and vinyl acetate is preferred as a vinyl ester, while N-vinyl-2-pyrrolidone is preferred as a vinyl amide. One such monomer (third monomer component) may be used or two or more may be used in combination.

When the acrylic pressure-sensitive adhesive is a copolymer of a (meth)acrylic acid alkyl ester (first monomer component) and a vinyl monomer having functional groups capable of contributing to a crosslinking reaction (second monomer component), the (meth)acrylic acid alkyl ester and vinyl monomer having functional groups capable of contributing to a crosslinking reaction are preferably blended and copolymerized at a weight ratio of 99 to 85 parts of (meth)acrylic acid alkyl ester per 1 to 15 parts of vinyl monomer having functional groups capable of contributing to a crosslinking reaction, or more preferably at a weight ratio of 99 to 90 parts per 1 to 10 parts.

When the acrylic pressure-sensitive adhesive is a copolymer of a (meth)acrylic acid alkyl ester (first monomer component), a vinyl monomer having functional groups capable of contributing to a crosslinking reaction (second monomer component) and another monomer (third monomer component), the (meth)acrylic acid alkyl ester, vinyl monomer having functional groups capable of contributing to a crosslinking reaction and other monomer are preferably blended and copolymerized at a weight ratio of 40 to 94 parts of (meth)acrylic acid alkyl ester, 1 to 15 parts of vinyl monomer having functional groups capable of contributing to a crosslinking reaction and 5 to 50 parts of other monomer, or more preferably at a weight ratio of 50 to 89 parts, 1 to 10 parts and 10 to 40 parts, respectively.

The polymerization reaction can be accomplished by known methods without any particular limitations, but one example is a method in which a polymerization initiator (such as benzoyl peroxide, azobisisobutyronitrile or the like) is added to the aforementioned monomers, which are then reacted for 5 to 48 hours at 50 to 70°C in a solvent (such as ethyl acetate).

Particularly desirable acrylic pressure-sensitive adhesives in the present invention include 2-ethylhexyl acrylate ester/acrylic acid/N-vinyl-2-pyrrolidone copolymer, 2-ethylhexyl acrylate ester/2-hydroxyethyl acrylate ester/vinyl acetate copolymer, 2-ethylhexyl acrylate ester/acrylic acid copolymer and the like for example, and 2-ethylhexyl acrylate ester/acrylic acid/N-vinyl-2-pyrrolidone copolymer is especially desirable.

The glass transition temperature of the acrylic pressure-sensitive adhesive in the present invention differs depending on the copolymer composition, but from the standpoint of adhesiveness of the percutaneously absorbable preparation it should normally be -100°C to -10°C, or preferably -90°C to -20°C.

In the percutaneously absorbable preparation of the present invention, a liquid plasticizer may be included in the pressure-sensitive adhesive layer in order to contribute softness to the pressure-sensitive adhesive layer and reduce the pain and skin irritation caused by skin adhesiveness when the percutaneously absorbable preparation is peeled off the skin. This liquid plasticizer may be any that is liquid at room temperature, exhibits a plasticizing effect and is compatible with the adhesive polymers making up the pressure-sensitive adhesive, without limitation, but is preferably one that improves the percutaneous absorbability and storage stability of donepezil. It can also be compounded with the aim of further improving the solubility and the like of the donepezil in the pressure-sensitive adhesive. Examples of such liquid plasticizers include fatty acid alkyl esters (for example, esters of lower monohydric alcohols with 1 to 4 carbon atoms with saturated or unsaturated fatty acids with 12 to 16 carbon atoms); saturated or unsaturated fatty acids with 8 to 10 carbon atoms (for example, caprylic acid (octanoic acid, C8), pelargonic acid (nonanoic acid, C9), capric acid (decanoic acid, C10), lauric acid (C12), etc.); ethylene glycol, diethylene glycol, triethylene glycol, polyethylene glycol, propylene glycol, polypropylene glycol and other glycols; olive oil, castor oil, squalene, lanoline and other oils and fats; ethyl acetate, ethyl alcohol, dimethyl decyl sulfoxide, decyl methyl sulfoxide, dimethyl sulfoxide, dimethyl formamide, dimethyl acetamide, dimethyl lauryl amide, dodecyl pyrrolidone, isosorbitol, oleyl alcohol and other organic solvents; liquid surfactants; diisopropyl adipate, phthalic acid esters, diethyl sebacate and other plasticizers; and liquid paraffin and other hydrocarbons and the like. Other examples include ethoxylated stearyl alcohol, glycerin esters (those liquid at room temperature), isotridecyl myristate, N-methylpyrrolidone, ethyl oleate, oleic acid, diisopropyl adipate, octyl palmitate, 1,3-propandiol, glycerin and the like. Of these, a fatty acid alkyl ester, saturated fatty acid, hydrocarbon or organic solvent is preferred from the standpoint of stability of the preparation, and a fatty acid alkyl ester is especially preferred. One such liquid plasticizer may be used alone or two or more may be used in combination.

Of these, when an acrylic pressure-sensitive adhesive is used for the pressure-sensitive adhesive the liquid plasticizer should be a fatty acid alkyl ester for purposes of compatibility and the like with the acrylic pressure-sensitive adhesive, and should more preferably be an ester of a lower monohydric alcohol having 1 to 4 carbon atoms with a saturated or unsaturated fatty acid having 12 to 16 carbon atoms. This saturated or unsaturated fatty acid having 12 to 16 carbon atoms is preferably a saturated fatty acid, and the lower monohydric alcohol having 1 to 4 carbon atoms may be either straight-chain or branched. Desirable examples of fatty acids with 12 to 16 carbon atoms include lauric acid (C12), myristic acid (C14) and palmitic acid (C16), while desirable examples of lower monohydric alcohols with 1 to 4 carbon atoms include isopropyl alcohol, ethyl alcohol, methyl alcohol, and propyl alcohol. Specific examples of particularly desirable fatty acid alkyl esters include isopropyl myristate, ethyl laurate, and isopropyl palmitat.

When using a fatty acid alkyl ester, a fatty acid having 8 to 10 carbon atoms and/or glycerin may be used in combination with the fatty acid alkyl ester in order to improve the percutaneous absorbability of the donepezil.

The blend amount of the liquid plasticizer in the present invention is preferably 10 to 160 parts by weight or more preferably 40 to 150 parts by weight per 100 parts by weight of the pressure-sensitive adhesive. If the blend amount is less than 10 parts by weight, the pressure-sensitive adhesive layer may not be sufficiently plasticized to produce the desired softness or reduce skin irritancy, while if the amount exceeds 160 parts by weight, the liquid plasticizer will not be retained in the pressure-sensitive adhesive even by the cohesive force of the adhesive, resulting in blooming on the surface of the pressure-sensitive adhesive layer and weakening the adhesive force to the point that the preparation may become detached from the skin surface during use.

In the percutaneously absorbable preparation of the present invention, crosslinking is accomplished by a known chemical crosslinking process (by addition of a crosslinking agent or the like) or physical crosslinking process (exposure to ultraviolet rays or gamma rays or other electron rays or the like) as discussed above, and this crosslinking process may be one commonly used in the technical field. Crosslinking by addition of a crosslinking agent is preferred because it has less effect on the drug.

In the case of a chemical crosslinking process using a crosslinking agent, the crosslinking agent is not particularly limited as long as crosslink formation is not impeded by the donepezil and the like, and examples include peroxides (such as benzoyl peroxide (BPO)), metal oxides (such as magnesium metasilicate aluminate), polyfunctional isocyanate compounds, organic metal compounds (such as zirconium alaninate, zinc alaninate, zinc acetate, glycine ammonium zinc, titanium compounds), metal alcoholates (such as tetraethyl titanate, tetraisopropyl titanate, aluminum isopropylate, aluminum secbutyrate) and metal chelate compounds (such as dipropoxy bis(acetylacetonate) titanium, tetraoctylene glycol titanium, aluminum isopropylate, ethylacetoacetate aluminum diisopropylate, aluminum tris(ethylacetoacetate) and aluminum tris(acetylacetonate)). Of these, a peroxide, metal oxide, organic metal compound, metal alcoholate or metal chelate compound is preferred and a metal alcoholate or metal chelate compound is more preferred from the standpoint of efficiently forming crosslink in the presence of donepezil and/or donepezil hydrochloride, while a metal chelate compound is best for easily obtaining crosslinked structures with a suitable crosslinking density. Of the metal chelate compounds, ethylacetoacetate aluminum diisopropylate is particularly desirable. One such crosslinking agent may be used or two or more may be used in combination.

The blend amount of the crosslinking agent differs depending on the type of crosslinking agent and pressure-sensitive adhesive, but is normally 0.1 to 0.6 parts by weight or preferably 0.15 to 0.5 parts by weight per 100 parts by weight of pressure-sensitive adhesive. Below 0.1 parts by weight there are too few crosslinking points to contribute sufficient cohesiveness to the pressure-sensitive adhesive layer, posing a risk of adhesive residue and strong skin irritancy due to cohesive failure during peeling, while above 0.6 parts by weight there is more cohesiveness but sufficient skin adhesiveness may not be obtained. Moreover, there may be skin irritation caused by a residue of unreacted crosslinking agent.

Chemical crosslinking treatment can be accomplished by first adding the crosslinking agent and then heating at or above the crosslinking reaction temperature, and the heating temperature in this case is selected appropriately according to the type of crosslinking agent but is preferably 60°C to 90°C or more preferably 60°C to 80°C. The heating time is preferably 12 to 96 hours or more preferably 24 to 72 hours.

In the percutaneously absorbable preparation of the present invention it is important that the crosslinked pressure-sensitive adhesive layer contain a metal chloride together with the donepezil and/or donepezil hydrochloride. When a metal chloride is included in the pressure-sensitive adhesive layer, there is less loss of cohesiveness of the pressure-sensitive adhesive layer when the percutaneously absorbable preparation is on human skin, and less likelihood of cohesive failure when the pressure-sensitive adhesive layer is peeled off.

This metal chloride is not particularly limited but may be a chloride of an alkali metal such as sodium or potassium; a chloride of an alkaline earth metal such as calcium or magnesium; or aluminum chloride, stannous chloride, or ferric chloride. From the standpoint of stability and controlling loss of cohesiveness of the pressure-sensitive adhesive layer, sodium chloride, calcium chloride, aluminum chloride, stannous chloride or ferric chloride is preferred, sodium chloride or calcium chloride is more preferred, and sodium chloride is especially preferred. Any of these may be used alone or two or more may be used in combination.

In general, the particle diameter of a metal chloride is classified into primary particle diameter and secondary particle diameter. When a metal chloride is observed under an optical microscope or electron microscope, the primary particles appear as small particles, while the secondary particles appear as large particles formed by aggregation of multiple primary particles. In these Specifications, the maximum particle diameter of each is given as the primary particle diameter and secondary particle diameter. These particle diameters of the metal chloride are not particularly limited, but smaller particle diameters are desirable from the standpoint of handling during manufacture and good skin feel due to smoothness of the affixed surface of the pressure-sensitive adhesive layer. Consequently, the secondary particle diameter of the metal chloride is preferably no more than 300 µm or more preferably no more than 250 µm or still more preferably no more than 200 µm. The minimum secondary particle diameter is not particularly limited but is preferably 1 µm or more. If the secondary particle diameter is less than 1 µm, the primary particle diameter of the metal chloride will be even smaller, potentially detracting from handling during manufacture due to particle scattering.

The secondary particles of the metal chloride are present in various forms such as flat, and amorphous in the pressure-sensitive adhesive layer. Unpredictably, as long as the secondary particle diameter of the metal chloride is the aforementioned preferred diameter, even if the secondary particle diameter of the metal chloride is larger than the thickness of the pressure-sensitive adhesive layer, the smoothness of the adhesive surface of the pressure-sensitive adhesive layer will probably not be affected, and there will be no problems with appearance of the preparation, because the particles are thoroughly accommodated in the pressure-sensitive adhesive layer. As discussed below, if the support is a relatively flexible support comprising paper, woven fabric or nonwoven fabric on the adhesive side, some of the particles of metal chloride will be embedded in the support, so that even if the secondary particle diameter of the metal chloride is larger than the thickness of the pressure-sensitive adhesive layer, a highly smooth patch surface can be easily obtained. If the support lacks relative flexibility, the secondary particle diameter of the metal chloride should preferably be no larger than the thickness of the pressure-sensitive adhesive layer in order to efficiently obtain a highly smooth patch surface, and should be more preferably 1/2 or less or still more preferably 1/3 or less of the thickness of the pressure-sensitive adhesive layer.

When the metal chloride is produced by neutralizing donepezil hydrochloride with an inorganic base containing a metal during the process of forming the pressure-sensitive adhesive layer as discussed below, the aforementioned preferred secondary particle diameter can be easily reproduced.

The particle diameter (primary particle diameter, secondary particle diameter) of the metal chloride in the percutaneously administered preparation of the present invention is a value measured by the following measurement methods.
The adhesive surface of the pressure-sensitive adhesive layer of the percutaneously administered preparation (sample) is observed with the naked eye (if the preparation includes a release sheet, the release sheet is peeled off and the adhesive surface of the exposed pressure-sensitive adhesive layer is observed with the naked eye). Next, the sample is set on an optical microscope stage with the adhesive surface of the pressure-sensitive adhesive layer facing up, and at least 20 particles, starting from those that appear largest, are photographed together with a scale under the optical microscope. If primary particles are being measured at least 20 primary particles only are photographed, while if secondary particles are being measured at least 20 secondary particles only are photographed. Of the photographed primary particles or secondary particles, the particle diameter of the particle judged to have the largest diameter according to the scale is taken as the primary particle diameter or secondary particle diameter. "Particle diameter" here means the diameter of a circle drawn around the projected image of the particle (circumscribed circle diameter). The "circumscribed circle diameter" is known as a convenient measure of particle diameter in microscopic particle size measurement, and is described for example in Funtai-Riron to Oyo-(Theory and Application) (Revised Second Edition, page 55, May 12, 1979, Maruzen).

The blend amount of the metal chloride is preferably 0.1 to 20 parts by weight or more preferably 1 to 15 parts by weight or still more preferably 3 to 10 parts by weight based on 100 parts by weight of the pressure-sensitive adhesive. When this amount is below 0.1 parts by weight, it may be insufficient to control loss of cohesiveness of the pressure-sensitive adhesive layer, while if it is over 20 parts by weight, it will have a control effect but may not be uniformly dispersed in the pressure-sensitive adhesive (adhesive polymer), detracting from the appearance of the preparation.

Because donepezil hydrochloride is more stable than donepezil, and because donepezil's crystal polymorphism makes it difficult to handle, donepezil hydrochloride is preferred over donepezil from the standpoint of handling. However, the free form of donepezil has better percutaneous absorbability than donepezil hydrochloride. To exploit both of these advantages, the metal chloride of the present invention may be a salt produced by neutralizing donepezil hydrochloride with an inorganic base containing a metal during formation of the pressure-sensitive adhesive layer. This salt is a metal chloride produced for example when donepezil hydrochloride is neutralized by mixing and agitating it in a solvent together with an inorganic base containing a metal, such as sodium hydroxide. In this way, a drug-containing liquid comprising a metal chloride can be prepared without the addition of a metal chloride, and using this drug-containing liquid comprising a metal chloride, the metal chloride and donepezil are present together in the final pressure-sensitive adhesive layer. After the metal chloride has been produced by mixing and agitating donepezil chloride in a solvent together with an inorganic base containing a metal, a metal chloride can also be further added to the resulting drug (donepezil)-containing liquid. Examples of inorganic bases containing metals include sodium hydroxide, potassium hydroxide, calcium hydroxide, magnesium hydroxide, sodium hydrogencarbonate, potassium hydrogencarbonate, sodium carbonate, potassium carbonate and other inorganic bases of alkali metals or alkaline earth metals, but a hydroxide of an alkali metal or alkaline earth metal is preferred from the standpoint of avoiding by-products, and sodium hydroxide, calcium hydroxide and magnesium hydroxide are especially preferred, with sodium hydroxide being most desirable.

In the percutaneously absorbable preparation of the present invention, antioxidants, various pigments, various fillers, stabilizers, drug solubilizers, drug dissolution inhibitors and other additives can be added as necessary to the pressure-sensitive adhesive layer. These stabilizers or antioxidants are not particularly limited, but 2-mercaptobenzimidazole, ascorbic acid, ascorbic acid palmitate, sodium metabisulfite, sodium sulfite, sodium bisulfite or the like is preferred, and one of these may be used or two or more may be combined. The content of these stabilizers or antioxidants in the pressure-sensitive adhesive layer is preferably about 0.0005 to 5 wt% or more preferably 0.001 to 3 wt% or still more preferably 0.01 to 1 wt% of the total pressure-sensitive adhesive layer.

In the percutaneously absorbable preparation of the present invention, the thickness of the pressure-sensitive adhesive layer is preferably 20 to 300 µm or more preferably 30 to 300 µm or still more preferably 50 to 300 µm. If the pressure-sensitive adhesive layer is less than 20 µm thick it will be difficult to obtain sufficient adhesive force, to include an effective amount of donepezil and to accommodate the secondary particles of the metal chloride, while if the pressure-sensitive adhesive layer is more than 300 µm thick it may be difficult to coat.

The percutaneously absorbable preparation of the present invention normally has a support, a pressure-sensitive adhesive layer and a release sheet. That is, the percutaneously absorbable preparation of the present invention has a structure comprising the aforementioned pressure-sensitive adhesive layer laminated on at least one side of a support, with the adhesive surface of the pressure-sensitive adhesive layer (the side opposite the side which is laminated on the support) being preferably covered and protected by a release sheet until just before use. A silicone, fluorine, wax or other back treatment may also be applied to the support, and the preparation can then be made into a roll without a release sheet.

The support is not particularly limited but is preferably one that does not allow the donepezil in the pressure-sensitive adhesive layer to pass through the support and be lost through the back, reducing the content of the drug (namely, a material that is impermeable to donepezil and the like), and is also preferably one that does not allow the donepezil and the liquid plasticizer to pass through the support and be lost from the back when a liquid plasticizer is contained in the pressure-sensitive adhesive layer as discussed below, reducing the content of the drug (namely, a material that is impermeable to the liquid plasticizer and donepezil).

Specific examples include polyester (such as polyethylene terephthalate (PET) or the like), nylon, polyvinylchloride, polyethylene, polypropylene, ethylene-vinyl acetate copolymer, polytetrafluoroethylene, ionomer resin and other single films, metal foil, and laminate films obtained by laminating two or more such films. Of these, the support is preferably a laminate film obtained by laminating a nonporous film consisting of one of the aforementioned materials with a porous film as described below in order to improve the adhesiveness (anchoring properties) of the support with the pressure-sensitive adhesive layer, with the pressure-sensitive adhesive layer preferably being formed on the porous film side.

This porous film is not particularly limited and may be any that improves anchorage properties with the pressure-sensitive adhesive layer, and examples include paper, woven fabrics, nonwoven fabrics (such as polyester (for example, polyethylene terephthalate (PET)) nonwoven fabric), and films obtained by mechanic perforation of the aforementioned films (such as polyester, nylon, saran, polyethylene, polypropylene, ethylene-vinyl acetate copolymer, polyvinylchloride, ethylene-ethyl acrylate copolymer, polytetrafluoroethylene, metal foil, polyethylene terephthalate and other single films and laminate films obtained by laminating one or two or more such films), and paper, nonwoven fabrics and woven fabrics (for example, polyester nonwoven fabric, and polyethylene terephthalate nonwoven fabric) are preferred from the standpoint of flexibility of the support. For purposes of improved anchorage properties and flexibility of the pressure-sensitive adhesive layer, the thickness of the porous film is normally 10 to 500 µm, and in the case of a thin percutaneously absorbable preparation such as a plaster or adhesive tape, it is normally about 1 to 200 µm. In the case of woven fabrics and nonwoven fabrics, the weight should preferably be 5 to 30 g/m² for purposes of improving anchorage properties.

The thickness of the support for the percutaneously absorbable preparation of the present invention is not particularly limited but is preferably 2 to 200 µm or more preferably 10 to 50 µm. Below 2 µm the handling properties, such as self-supporting properties may be adversely affected, while above 200 µm it may feel unpleasant (stiff), detracting from compliance.

The release sheet is not particularly limited, and a known release sheet may be used. Specifically, the release sheet may be a release sheet comprising a release agent layer of a release agent formed on the surface of a release sheet base, or a plastic film which itself has good release properties, or a release sheet comprising a release layer of such a plastic film material with good release properties formed on the surface of a release sheet base. The release sheet may have a release surface on only one side of the base, or on both sides.

In this release sheet, the release agent is not particularly limited, and examples include long-chain alkyl group-containing polymers, silicone polymers (silicone release agents), fluorine polymers (fluorine release agents) and other release agents. The base for the release sheet may be of polyethylene terephthalate (PET) film, polyimide film, polypropylene film, polyethylene film, polycarbonate film, polyester (other than PET) film or other plastic film or metal-deposited plastic film comprising metal deposited on one of these films; or of Japanese paper, paper, kraft paper, glassine paper, fine paper (bookpaper) or other paper; or of nonwoven fabric, fabric or other fibrous material; or of metal foil or the like.

Examples of plastic films that themselves have good release properties include polyethylene (low-density polyethylene, linear low-density polyethylene, etc.), polypropylene and ethylene-propylene copolymer and other ethylene-α-olefin copolymers (block copolymers or random copolymers), as well as polyolefin films formed from polyolefin resins consisting of mixtures of these, and Teflon^{™} films for example.

The release layer formed on the surface of the aforementioned release sheet base can be formed laminating or coating the material of the aforementioned plastic film with good release properties on the aforementioned release sheet base.

The thickness (overall thickness) of the release sheet is not particularly limited but is normally 200 µm or less or preferably 25 to 100 µm.

The method for manufacturing the percutaneously absorbable preparation of the present invention is not particularly limited, but for example it is preferably manufactured by the manufacturing methods of (i) through (iii) below.

(i) A drug-containing pressure-sensitive adhesive liquid is prepared by dissolving or dispersing donepezil and/or donepezil hydrochloride in a solvent together with a pressure-sensitive adhesive and a liquid plasticizer and other additives as necessary, this is mixed and agitated with a metal chloride or a dispersion of a metal chloride dispersed in a solvent such as ethanol, and a crosslinking agent is added. The resulting mixture is applied to one side of a support or to the release-treated surface of a release sheet and dried to form a pressure-sensitive adhesive layer, a release sheet or support is affixed to this pressure-sensitive adhesive layer, and aging is performed for purposes of crosslinking.

(ii) When using a liquid plasticizer, the donepezil and/or donepezil hydrochloride, pressure-sensitive adhesive, liquid plasticizer and other additives as necessary are mixed and agitated as is, this mixture is then mixed and agitated with a metal chloride or a dispersion of a metal chloride dispersed in a solvent such as ethanol, and a crosslinking agent is added to obtain a mixture which is then applied to one side of a support or to the release-treated surface of a release sheet and dried to form a pressure-sensitive adhesive layer, a release sheet or support is affixed to the pressure-sensitive adhesive layer, and aging is performed for purposes of crosslinking.

(iii) A dispersion of a metal chloride dispersed in a solvent such as ethanol is mixed and agitated together with donepezil and/or donepezil hydrochloride to prepare a drug-containing solution. When using donepezil hydrochloride as the drug, the donepezil hydrochloride, a metal hydroxide and a solvent such as ethanol as necessary are mixed and agitated to neutralize the donepezil hydrochloride and produce a metal chloride, and a metal chloride is added as necessary and mixed to obtain a drug-containing solution. A pressure-sensitive adhesive-containing liquid comprising a pressure-sensitive adhesive dissolved or dispersed in a solvent together with a liquid plasticizer and other additives as necessary is also prepared, or else when using a liquid plasticizer, an adhesive-containing liquid is prepared by mixing and agitating the pressure-sensitive adhesive, liquid plasticizer and other additives as necessary as is. Next, the aforementioned drug-containing solution is added to the adhesive-containing solution and agitated, a crosslinking agent is added, and the resulting mixture is applied to one side of a support or to the release-treated surface of a release sheet and dried to form a pressure-sensitive adhesive layer, a release sheet or support is affixed to the pressure-sensitive adhesive layer, and aging is performed for purposes of crosslinking.

In the methods of (i) through (iii) above, the solvent for dissolving or dispersing the pressure-sensitive adhesive may be ethyl acetate, toluene, hexane, 2-propanol, methanol, ethanol, or water for example. These may also be used to adjust the viscosity after addition of the crosslinking agent.

Because the method of (iii) above donepezil hydrochloride, which is easier to handle than donepezil, is used to produce a preparation in which most of the drug in the pressure-sensitive adhesive layer is donepezil (free form), which has better percutaneous absorbability, it is possible to reliably manufacture a preparation with excellent percutaneous absorbability.

The form of the percutaneously absorbable preparation of the present invention is not particularly limited, and may be a tape form, or sheet form for example. The dosage of the percutaneously absorbable preparation of the present invention differs depending on the types and amounts of pressure-sensitive adhesive and liquid plasticizer used, the age, body weight and symptoms of the patient, but in the case of an adult it is normally desirable to apply a percutaneously absorbable preparation containing 2 to 150 mg of donepezil or donepezil hydrochloride to 5 to 120 cm² of skin once every 7 days to once a day.

### [Examples]

The present invention is explained in detail below using examples, but the present invention is not limited by these examples. Parts in the text below all signify parts by weight.

### 1. Preparation of acrylic pressure-sensitive adhesive

### (1) Acrylic pressure-sensitive adhesive solution A

75 parts of 2-ethylhexyl acrylate, 22 parts of N-vinyl-2-pyrrolidone, 3 parts of acrylic acid and 0.2 parts of azobisisobutyronitrile were solution polymerized in ethyl acetate at 60°C in an inactive gas atmosphere to obtain pressure-sensitive adhesive solution A (pressure-sensitive adhesive solids: 28 wt%).

### (2) Acrylic pressure-sensitive adhesive solution B

95 parts of 2-ethylhexyl acrylate, 5 parts of acrylic acid and 0.2 parts of benzoyl peroxide were solution polymerized in ethyl acetate at 60°C in an inactive gas atmosphere to obtain acrylic pressure-sensitive adhesive solution B (pressure-sensitive adhesive solids: 35 wt%).

### 2. Percutaneously absorbable preparations produced using donepezil

### Example 1

40 parts of isopropyl myristate, 8 parts of donepezil and an amount of acrylic pressure-sensitive adhesive solution A containing 51 parts of pressure-sensitive adhesive solids were mixed and agitated in a container until homogenous. 1 part of sodium chloride with a primary particle diameter of about 10 µm and a secondary particle diameter of about 50 µm dispersed in ethanol was then added to this mixture and agitated, and ethylacetoacetate aluminum diisopropylate was added in the amount of 0.4 parts per 100 parts of pressure-sensitive adhesive solids, the viscosity was adjusted with ethyl acetate, and this liquid was applied to a dried thickness of 60 µm to a PET film (75 µm thick) and dried to form a pressure-sensitive adhesive layer which was then laminated with a silicone-treated PET film (12 µm thick) and aged for 48 hours at 70°C (crosslinking treatment of pressure-sensitive adhesive layer) to obtain a percutaneously absorbable preparation of donepezil.

### Example 2

A percutaneously absorbable preparation of donepezil was prepared as in Example 1 except that the amount of acrylic pressure-sensitive adhesive solution A was changed to one with an adhesive solids content of 49 parts, while the added amount of sodium chloride dispersed in ethanol was changed to 3 parts.

### Example 3

A percutaneously absorbable preparation of donepezil was prepared as in Example 1 except that the amount of acrylic pressure-sensitive adhesive solution A was changed to one with an adhesive solids content of 47 parts, while the added amount of sodium chloride dispersed in ethanol was changed to 5 parts.

### Example 4

40 parts of isopropyl myristate, 8 parts of donepezil and an amount of acrylic pressure-sensitive adhesive solution B containing 49 parts of pressure-sensitive adhesive solids were mixed and agitated in a container until homogenous. 3 parts of the same sodium chloride as in Example 1 dispersed in ethanol were then added to this mixture and agitated, and ethylacetoacetate aluminum diisopropylate was added in the amount of 0.4 parts per 100 parts of pressure-sensitive adhesive solids, the viscosity was adjusted with ethyl acetate, and a percutaneously absorbable preparation of donepezil was then obtained by the same operations as in Example 1.

### Comparative Example 1

40 parts of isopropyl myristate, 8 parts of donepezil and an amount of acrylic pressure-sensitive adhesive solution A containing 52 parts of pressure-sensitive adhesive solids were mixed and agitated in a container until homogenous, ethylacetoacetate aluminum diisopropylate was added in the amount of 0.4 parts per 100 parts of pressure-sensitive adhesive solids, the viscosity was adjusted with ethyl acetate, and a percutaneously absorbable preparation of donepezil was then obtained by the same operations as in Example 1.

### Comparative Example 2

A percutaneously absorbable preparation of donepezil was prepared as in Comparative Example 1 except that the amount of acrylic pressure-sensitive adhesive solution A containing 52 parts of pressure-sensitive adhesive solids was changed to an amount of acrylic pressure-sensitive adhesive solution B containing 52 parts of pressure-sensitive adhesive solids.

### 3. Experiment 1

The following cohesive failure evaluation test was performed on the percutaneously absorbable preparations prepared in the various Examples and Comparative Examples above.

### [Cohesive failure evaluation test]

The cohesiveness of the pressure-sensitive adhesive layer was evaluated by the following methods as though the preparation were actually applied to the skin. 10 cm² (3.16 cm x 3.16 cm) samples were punched out of the resulting preparations. To replicate the environment of the skin surface, 5 mL of physiological saline containing 0.4 wt% lactic acid was added to a glass Petri dish, the separator was peeled off and the sample was soaked (floated) with the adhesive surface down. One day (24 hours) after the start of soaking, the preparation was removed, the surface was dried, and it was evaluated by finger touch. The results are shown in Table 1.

**[Table 1]**

| | COHESIVE FAILURE EVALUATION |
|---|---|
| Example 1 | No cohesive failure |
| Example 2 | No cohesive failure |
| Example 3 | No cohesive failure |
| Example 4 | No cohesive failure |
| Comparative Example 1 | Cohesive failure |
| Comparative Example 2 | Cohesive failure |

It can be seen from the results of Table 1 that a preparation resistant to cohesive failure of the pressure-sensitive adhesive layer during skin contact can be obtained by including a metal chloride in the pressure-sensitive adhesive layer together with donepezil.

### 4. Percutaneously absorbable preparations produced using donepezil hydrochloride

### Example 5

40 parts of isopropyl myristate and an amount of acrylic pressure-sensitive adhesive solution A containing 50.13 parts of pressure-sensitive adhesive solids were mixed and agitated in a container until homogenous, and 9 parts of donepezil hydrochloride and 0.87 parts of a 10 wt% ethanol solution of sodium hydroxide were mixed and agitated in a separate container. This drug-containing mixture was added to the aforementioned mixture of pressure-sensitive adhesive solution A and isopropyl myristate and agitated, ethylacetoacetate aluminum diisopropylate was added in the amount of 0.4 parts per 100 parts of pressure-sensitive adhesive solids, the viscosity was adjusted with ethyl acetate, and this liquid was applied to a dried thickness of 60 µm to PET film (75 µm thick), dried, laminated to a silicone release-treated PET film (12 µm thick), and aged for 48 hours at 70°C to obtain a percutaneously absorbable preparation.

### Example 6

A percutaneously absorbable preparation was obtained as in Example 5 except that the amount of acrylic pressure-sensitive adhesive solution A was changed to one with an adhesive solids content of 46.85 parts, the amount of donepezil hydrochloride was changed to 12 parts, and the amount of the 10 wt% ethanol solution of sodium hydroxide was changed to 1.15 parts.

### Example 7

A percutaneously absorbable preparation was obtained as in Example 5 except that the amount of acrylic pressure-sensitive adhesive solution A was changed to one with an adhesive solids content of 43.56 parts, the amount of donepezil hydrochloride was changed to 15 parts, and the amount of the 10 wt% ethanol solution of sodium hydroxide was changed to 1.44 parts.

### Example 8

A percutaneously absorbable preparation was obtained as in Example 5 except that pressure-sensitive adhesive solution B containing 50.13 parts pressure-sensitive adhesive solids was used instead of pressure-sensitive adhesive solution A containing 50.13 parts of pressure-sensitive adhesive solids.

### Example 9

A percutaneously absorbable preparation was obtained as in Example 5 except that the amount of acrylic pressure-sensitive adhesive solution A was changed to an amount giving 60.13 parts of pressure-sensitive adhesive solids, while the amount of isopropyl myristate was changed to 30 parts.

### Example 10

A percutaneously absorbable preparation was obtained as in Example 5 except that the amount of acrylic pressure-sensitive adhesive solution A was changed to an amount giving 40.13 parts of pressure-sensitive adhesive solids, while the amount of isopropyl myristate was changed to 50 parts.

### Comparative Example 3

40 parts of isopropyl myristate and an amount of acrylic pressure-sensitive adhesive solution A containing 49.42 parts of pressure-sensitive adhesive solids were mixed and agitated in a container until homogenous, and 9 parts of donepezil hydrochloride and 1.58 parts of a 10 wt% 2-propanol solution of n-butylamine were mixed and agitated in a separate container. This drug-containing mixture was added to the aforementioned mixture of pressure-sensitive adhesive solution A and isopropyl myristate and agitated, ethylacetoacetate aluminum diisopropylate was added in the amount of 0.4 parts per 100 parts of pressure-sensitive adhesive solids, the viscosity was adjusted with ethyl acetate, and a percutaneously absorbable preparation was prepared by the same operations as in Example 5 using this liquid.

### Comparative Example 4

40 parts of isopropyl myristate and an amount of acrylic pressure-sensitive adhesive solution B containing 49.09 parts of pressure-sensitive adhesive solids were mixed and agitated in a container until homogenous, and 9 parts of donepezil hydrochloride and 1.91 parts of a 10 wt% 2-propanol solution of N,N-dimethyldiamine were mixed and agitated in a separate container. This drug-containing mixture was added to the aforementioned mixture of pressure-sensitive adhesive solution B and isopropyl myristate and agitated, ethylacetoacetate aluminum diisopropylate was added in the amount of 0.4 parts per 100 parts of pressure-sensitive adhesive solids, the viscosity was adjusted with ethyl acetate, and a percutaneously absorbable preparation was prepared by the same operations as in Example 5 using this liquid.

### Comparative Example 5

40 parts of isopropyl myristate and an amount of acrylic pressure-sensitive adhesive solution B containing 46.86 parts of pressure-sensitive adhesive solids were mixed and agitated in a container until homogenous, and 9 parts of donepezil hydrochloride and 4.14 parts of a 10 wt% 2-propanol solution of triisopropanolamine were mixed and agitated in a separate container. This drug-containing mixture was added to the aforementioned mixture of pressure-sensitive adhesive solution B and isopropyl myristate and agitated, ethylacetoacetate aluminum diisopropylate was added in the amount of 0.4 parts per 100 parts of pressure-sensitive adhesive solids, the viscosity was adjusted with ethyl acetate, and a percutaneously absorbable preparation was prepared by the same operations as in Example 5 using this liquid.

### 5. Experiment 2

The aforementioned cohesive failure evaluation test was performed on the percutaneously absorbable preparations prepared in Examples 5 through 10 and Comparative Examples 3 through 5 above. The results are given in Table 2 below.

**[Table 2]**

| | COHESIVE FAILURE EVALUATION |
|---|---|
| Example 5 | No cohesive failure |
| Example 6 | No cohesive failure |
| Example 7 | No cohesive failure |
| Example 8 | No cohesive failure |
| Example 9 | No cohesive failure |
| Example 10 | No cohesive failure |
| Comparative Example 3 | Cohesive failure |
| Comparative Example 4 | Cohesive failure |
| Comparative Example 5 | Cohesive failure |

It can be seen from the results of Table 2 that in the preparations of Examples 5 through 10, in which the pressure-sensitive adhesive layer is formed using a mixed solution of donepezil hydrochloride and an inorganic base containing a metal, cohesive failure of the pressure-sensitive adhesive layer during skin attachment was avoided as in the preparations of Examples 1 through 4, which contained donepezil and a metal chloride in the pressure-sensitive adhesive layer. By contrast, the preparations of Comparative Examples 3 through 5, in which the pressure-sensitive adhesive layer is formed using a mixed solution of donepezil hydrochloride and a basic compound containing no metal (n-butylamine, N,N-dimethyldiamine, triisopropanolamine), were prone to cohesive failure of the pressure-sensitive adhesive layer during skin attachment just like Comparative Examples 1 and 2, which contained no metal chloride together with donepezil in the pressure-sensitive adhesive layer. This confirms that a metal chloride is produced by a neutralization reaction during preparation of a mixed solution of donepezil hydrochloride and an inorganic base containing a metal, and that due to the presence of this metal chloride, a preparation is achieved which is resistant to cohesive failure of the pressure-sensitive adhesive layer during skin attachment just as if a metal chloride had been included in the pressure-sensitive adhesive layer together with donepezil.

### 6. Experiment 3

The primary particle diameter and secondary particle diameter of the sodium chloride of the preparations of Examples 1 through 10 were measured by the methods described previously. In the preparations of Examples 1 through 5 the sodium chloride had a primary particle diameter of about 10 µm and a secondary particle diameter of about 50 µm. In Examples 6 through 10 the sodium chloride had a primary particle diameter of between 2 and 3 µm in each case and a secondary particle diameter of about 20 µm in each case. The smoothness of the surface of the pressure-sensitive adhesive layer was evaluated with the naked eye in each example, and the skin feel was evaluated organoleptically by touching the surface of the pressure-sensitive adhesive layer with the fingers. Compared with Examples 1 through 5, Examples 6 through 10 tended to have better smoothness and skin feel.

### 7. Other preparation examples

### Example 11

A percutaneously absorbable preparation with the following composition was prepared according to the same manufacturing steps as the preparation of Example 5. The sodium metabisulfite and ascorbic acid in the pressure-sensitive adhesive layer were added before addition of the ethylacetoacetate aluminum diisopropylate.

### (A) Support

Laminate of PET nonwoven fabric (weight 12 g/m²)/PET film (2.0 µm)

### (B) Pressure-sensitive adhesive layer

Composition (based on weight of pressure-sensitive adhesive layer)

| | |
|---|---|
| Donepezil (free base) | 7.57 wt% |
| Acrylic pressure-sensitive adhesive A | 40.53 wt% |
| Isopropyl myristate | 50.00 wt% |
| Sodium chloride | 1.16 wt% |
| Sodium hydroxide | 0.01 wt% |
| Sodium metabisulfite | 0.50 wt% |
| Ascorbic acid | 0.05 wt% |
| Ethylacetoacetate aluminum diisopropylate | 0.18 wt% |
| (Total: | 100 wt%) |

### (C) Release sheet

75 µm-thick PET film with silicone release-treated surface

### Example 12

A percutaneously absorbable preparation with the following composition was prepared according to the same manufacturing steps as the preparation of Example 5. The support and release sheet were the same as those used in Example 11.

| | |
|---|---|
| Donepezil (free base) | 7.57 wt% |
| Acrylic pressure-sensitive adhesive A | 41.08 wt% |
| Isopropyl myristate | 50.00 wt% |
| Sodium chloride | 1.16 wt% |
| Sodium hydroxide | 0.01 wt% |
| Ethylacetoacetate aluminum diisopropylate | 0.18 wt% |
| (Total: | 100 wt%) |

## Claims

1. A percutaneously absorbable preparation comprising 2-[(1-benzyl-4-piperidinyl)methyl]-5,6-dimethoxyindan-1-on and/or its hydrochloride and a metal chloride in a pressure-sensitive adhesive layer, wherein the pressure-sensitive adhesive layer is crosslinked.

2. The percutaneously absorbable preparation according to Claim 1, wherein the pressure sensitive adhesive layer is crosslinked using a crosslinking agent.

3. The percutaneously absorbable preparation according to Claim 2, wherein the crosslinking agent is a metal alcoholate or metal chelate compound.

4. The percutaneously absorbable preparation according to any one of Claims 1 through 3, wherein the pressure-sensitive adhesive is an acrylic pressure-sensitive adhesive.

5. The percutaneously absorbable preparation according to any one of Claims 1 through 4, wherein the pressure-sensitive adhesive layer further comprises a liquid plasticizer.

6. The percutaneously absorbable preparation according to Claim 5, wherein the liquid plasticizer is a fatty acid alkyl ester produced by reacting a higher fatty acid having 12 to 16 carbon atoms with a lower monohydric alcohol having 1 to 4 carbon atoms.

7. The percutaneously absorbable preparation according to any one of Claims 1 through 6, wherein the metal chloride is at least one species selected from the group consisting of sodium chloride, calcium chloride, aluminum chloride, stannous chloride and ferric chloride.

8. The percutaneously absorbable preparation according to any one of Claims 1 through 6, wherein the metal chloride is sodium chloride.

9. The percutaneously absorbable preparation according to Claim 1, wherein the metal chloride is a salt produced by neutralizing a hydrochloride of 2-[(1-benzyl-4-piperidinyl)methyl]-5,6-dimethoxyindan-1-on with an inorganic base containing a metal.

10. The percutaneously absorbable preparation according to Claim 9, wherein the inorganic base containing a metal is an inorganic base containing an alkali metal or alkaline earth metal.

## Patentansprüche

1. Perkutan absorbierbare Zubereitung, die 2-[(1-Benzyl-4-piperidinyl)methyl]-5,6-dimethoxyindan-1-on und/oder dessen Hydrochlorid und ein Metallchlorid in einer druckempfindlichen Adhäsivschicht umfasst, worin die druckempfindliche Adhäsivschicht vernetzt ist.

2. Perkutan absorbierbare Zubereitung gemäß Anspruch 1, worin die druckempfindliche Adhäsivschicht unter Verwendung eines Vernetzungsmittels vernetzt ist.

3. Perkutan absorbierbare Zubereitung gemäß Anspruch 2, worin das Vernetzungsmittel ein Metallalkoholat oder eine Metallchelatverbindung ist.

4. Perkutan absorbierbare Zubereitung gemäß irgendeinem der Ansprüche 1 bis 3, worin das druckempfindliche Adhäsivmittel acrylisches druckempfindliches Adhäsivmittel ist.

5. Perkutan absorbierbare Zubereitung gemäß irgendeinem der Ansprüche 1 bis 4, worin die druckempfindliche Adhäsivschicht weiterhin einen flüssigen Plastifizierer umfasst.

6. Perkutan absorbierbare Zubereitung gemäß Anspruch 5, worin der flüssige Plastifizierer ein Fettsäure-Alkylester ist, der durch Umsetzung einer höheren Fettsäure mit 12 bis 16 Kohlenstoffatomen mit einem niederen einwertigen Alkohol mit 1 bis 4 Kohlenstoffatomen hergestellt wird.

7. Perkutan absorbierbare Zubereitung gemäß irgendeinem der Ansprüche 1 bis 6, worin das Metallchlorid mindestens eine Sorte ausgewählt aus der Gruppe bestehend aus Natriumchlorid, Kalziumchlorid, Aluminiumchlorid, Zinnchlorid und Eisenchlorid ist.

8. Perkutan absorbierbare Zubereitung gemäß irgendeinem der Ansprüche 1 bis 6, worin das Metallchlorid Natriumchlorid ist.

9. Perkutan absorbierbare Zubereitung gemäß Anspruch 1, worin das Metallchlorid ein Salz ist, das durch Neutralisieren eines Hydrochlorids von 2-[(1-Benzyl-4-piperidinyl)methyl]-5,6-dimethoxyindan-1-on mit einer anorganischen Base, die ein Metall enthält, hergestellt wird.

10. Perkutan absorbierbare Zubereitung gemäß Anspruch 9, worin die anorganische Base, die ein Metall enthält, eine anorganische Base ist, die ein Alkalimetall oder Erdalkalimetall enthält.

## Revendications

1. Préparation à absorption percutanée comprenant une 2-[(1-benzyl-4-pipéridinyl)méthyl]-5,6-diméthoxyindan-1-one et/ou son chlorhydrate et un chlorure de métal dans une couche d'adhésif sensible à la pression, dans laquelle la couche d'adhésif sensible à la pression est réticulée.

2. Préparation à absorption percutanée selon la revendication 1, dans laquelle la couche d'adhésif sensible à la pression est réticulée en utilisant un agent de réticulation.

3. Préparation à absorption percutanée selon la revendication 2, dans laquelle l'agent de réticulation est un composé alcoolate de métal ou chélate de métal.

4. Préparation à absorption percutanée selon l'une quelconque des revendications 1 à 3, dans laquelle l'adhésif sensible à la pression est un adhésif acrylique sensible à la pression.

5. Préparation à absorption percutanée selon l'une quelconque des revendications 1 à 4, dans laquelle la couche d'adhésif sensible à la pression comprend en outre un plastifiant liquide.

6. Préparation à absorption percutanée selon la revendication 5, dans laquelle le plastifiant liquide est un ester alkylique d'acide gras produit par réaction d'un acide gras supérieur ayant 12 à 16 atomes de carbone avec un alcool monohydrique inférieur ayant 1 à 4 atomes de carbone.

7. Préparation à absorption percutanée selon l'une quelconque des revendications 1 à 6, dans laquelle le chlorure de métal est au moins une espèce choisie parmi le groupe consistant en un chlorure de sodium, un chlorure de calcium, un chlorure d'aluminium, un chlorure stanneux et un chlorure ferrique.

8. Préparation à absorption percutanée selon l'une quelconque des revendications 1 à 6, dans laquelle le chlorure de métal est un chlorure de sodium.

9. Préparation à absorption percutanée selon la revendication 1, dans laquelle le chlorure de métal est un sel produit par neutralisation d'un chlorhydrate de 2-[(1-benzyl-4-pipéridinyl)méthyl]-5,6-diméthoxyindan-1-one avec une base inorganique contenant un métal.

10. Préparation à absorption percutanée selon la revendication 9, dans laquelle la base inorganique contenant un métal est une base inorganique contenant un métal alcalin ou un métal alcalinoterreux.
